(19) Europäisches Patentamt European Patent Office Office européen des brevets

(11) **EP 2 517 692 B1**

(12) **EUROPEAN PATENT SPECIFICATION**

(45) Date of publication and mention
of the grant of the patent:
**16.03.2016 Bulletin 2016/11**

(51) Int Cl.:
*A61K 8/31* (2006.01)   *A61K 8/46* (2006.01)
*A61Q 9/04* (2006.01)

(21) Application number: **11165166.7**

(22) Date of filing: **06.05.2011**

(54) **Hair removal method and kit**

Haarentfernungsverfahren und Kit

Procédé et kit de suppression des poils

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **28.04.2011  US 479876 P**

(43) Date of publication of application:
**31.10.2012  Bulletin 2012/44**

(73) Proprietor: **The Procter & Gamble Company
Cincinnati, OH 45202 (US)**

(72) Inventor: **Smith, Charles, Robert
Henley on Thames, Oxfordshire RG9 3EB (GB)**

(74) Representative: **Kohol, Sonia
Technical Centres Limited
Procter & Gamble Patent Department
Rusham Park
Whitehall Lane
Egham, Surrey TW20 9NW (GB)**

(56) References cited:
**EP-A2- 0 095 916      WO-A1-99/36031
WO-A1-2004/096164   CA-A1- 2 555 095
DE-A1- 10 156 297    US-A- 5 494 657**

EP 2 517 692 B1

**Description**

FIELD OF THE INVENTION

The present invention relates to a method and kit for depilation.

BACKGROUND OF THE INVENTION

Depilatory compositions are cosmetic hair removal formulations. They comprise keratin reducing agents, which attack the disulphide bonds in hair to weaken it, such that subsequent gentle scraping and/or wiping completes severance of the hair from the skin and effects hair removal. Commercially, the most common keratin reducing agents are thioglycolates, which are typically formulated at high pH. An unwanted side effect of chemical depilation is that the depilatory composition comes into contact with and must have a relatively long residence time on skin to achieve effective hair removal and this long residence time combined with the alkaline conditions needed for effective hair removal may give rise to skin irritation.

The above problem has been recognized in the art. Reference is made to US 4,401,663 and US 4,424,205 the disclosures of which are similar to one another. These documents teach to use certain compounds as topical analgesics and an example given of a situation in which it is suggested to use such materials is to reduce depilatory irritation. The carrier formulas disclosed to be suitable for formulation with the analgesics include lotions and creams.

Reference is also made to US 2004/0219118, which discloses treatment with a "lipophilic" material before application of a thioglycolate-based reactive depilatory compostion. Lipophilic materials exemplified in this patent application are oils, such as mineral oil. As shown hereinbelow, the present applicants have tested a range of lipophilic materials to determine their ability to prevent thioglycolate penetration and, thereby, their ability to reduce or prevent skin irritation Applicants have surprisingly found that oils, such as mineral oil, have no or a low ability to prevent thioglycolate penetration to the skin. There thus exists a need to develop a pre-treatment composition which better reduces skin irritation.

SUMMARY OF THE INVENTION

According to a first aspect of the invention, a method of removing hair from skin is provided, comprising the steps of:

(a) applying a protective composition to an area of skin on which unwanted hair is growing, wherein the protective composition comprises at least 75% petrolatum by weight of the protective composition, the protective composition having a cone penetration of 160 to 210;

(b) applying a depilatory composition to the area of skin to which the protective composition has been applied, the depilatory composition comprising a keratin reducing agent.

According to a second aspect of the invention, a depilatory kit is provided, comprising:

(a) a protective composition, the protective composition comprising at least 75% petrolatum by weight of the protective composition, the protective composition having a cone penetration of 160 to 210;

(b) a depilatory composition comprising an effective amount of a keratin reducing agent.

BRIEF DESCRIPTION OF THE DRAWINGS

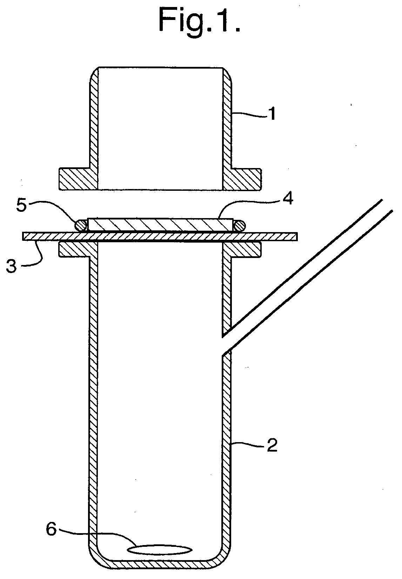

Fig.1. is a schematic view of a Franz Cell apparatus.

DETAILED DESCRIPTION OF THE INVENTION

The protective composition used in the method and comprised within the kit according to the invention comprises at least 75% petrolatum by weight of the protective composition, the protective composition having a cone penetration of 160 to 210. Applicants have found, as demonstrated by the Franz Cell data presented herein, that a composition having the defined cone penetration, which is a measure of hardness and comprising at least 75% petrolatum, inhibits, penetration by thioglycolic acid versus prior art compositions, such as the oils disclosed in US 2004/0219118 discussed above, to a surprising degree. A reduction of thioglycolic acid penetration of 45% or more according to the Franz Cell method may be shown to correlate to a significant and user-noticeable reduction in irritation. At the same time, applicants have established that the use of the present protective composition still permits hair weakening and destruction by the keratin reducing agent, so that reduced skin irritation can be achieved while removing hair. A cone penetration of less than 160 defines a protective composition which is too difficult to spread and will generally reduce hair removal efficacy and a cone penetration of above 210 defines a composition which is too thin to generate a coherent film that provides an effective barrier, resulting in an increase in irritation.

As used herein, the term "cone penetration" refers to cone penetration as measured by the method of ASTM Standard D937, 2007, "Standard Test Method for Cone Penetration of Petrolatum" ASTM International, West Conshohocken, PA, 2003, DOI: 10.1520/D0937-07, www.astm.org. This method was devised to measure petrolatum alone, but may be used to measure the cone penetration of the present compositions, given the high level of petrolatum comprised within them. For completeness, commercially available petrolatum has quoted cone penetration values in the range from 70 to 350. Cone penetration in the D937 ASTM standard state is measured as the number of 1/10th millimeters, so that, by way of example, a "200" value of cone penetration equates to a 20mm penetration.

Reference is also made to the following standard for the equipment needed (such as the cone and equipment specifications): ASTM Standard D217, 2010, " Standard Test Methods for Cone Penetration of Lubricating Grease" ASTM International, West Conshohocken, PA, 2003, DOI: 10.1520/D0217-10, www.astm.org.

Advantageously, the protective composition comprises at least 80%, preferably at least 90% and more preferably at least 95% petrolatum by weight of the protective compositon. While the present compositions may consist entirely of petrolatum (that is, they may be 100% petrolatum), it is preferred that they comprise at least a small proportion of cosmetic excipients, such as perfumes and dyes to make them more pleasant to use.

The protective composition may comprise skin active agents such as, but not limited to oil soluble vitamins, such as vitamin E derivatives, including vitamin E acetate and tocopherol nicotinate; oil-soluble vitamin A derivatives, such as retinyl palmitate; lanolin; ceramides; sterols and sterol esters; salicylic acid; camphor; eucalyptol; essential oils and mixtures thereof.

The protective composition used in the method and comprised within the kit according to the invention may comprise from 1% to 25%, preferably from 1.5% to 15%, more preferably from 3% to 10% of powder to provide a tactile and/or an optical signal to the user allowing them easily to establish to which areas of skin the protective composition has been applied and which not.

In order to have a user-perceptible tactile effect on skin, the powder comprised within the protective composition may advantageously have an average particle size from $0.5\mu m$ to $200\mu m$, preferably from $1\mu m$ to $130\mu m$, more preferably from $5\mu m$ to $30\mu m$.

In order to have a user-perceptible visual effect on skin, the powder comprised within the protective composition may advantageously have an average particle size from $0.05\mu m$ to $200\mu m$, preferably from $0.1\mu m$ to $130\mu m$, more preferably from $0.2\mu m$ to $60\mu m$.

The average particle size is measured using the laser diffraction method defined in ISO13320.

If it is desired to create a user-perceptible visual effect on skin, then, it may be advantageous to use a dye instead of or as well as the powder according to the invention. Any dye which is compatible with the protective composition may be employed and preferably the dye is soluble in wax.

Advantageously, at least some of the particles are coloured to contrast with the skin colour and render them visible to the naked eye. Highly advantageously, they are provided with a colour, such as a green or blue, which is not a human skin colour and may provide a strong contrast to the colour of skin. It is not essential that they be coloured, however, because the particles may provide a tactile signal to the user, whatever colour they are.

The particles may be comprised of any suitable material that may provide a tactile or visible signal to the user and is not otherwise unsuitable, for example because it is an irritant. Suitable materials for the particles may include metals; inorganic materials, such as metal salts and metal oxides, silica, mica and talc; organic materials, such as natural and synthetic polymers.

Exemplary materials from which the particles may be comprised include spherical silica, hydrated silica, silicone treated silica beads, mica, talc, iron oxide, titanium dioxide, zinc oxide, aluminium lake pigments, nylon 12 and nylon 6, polyethylene, starch (such as aluminium starch octenyl succinate), methylsilsequioxane resin, polymethylmethacrylates,; crosslinked polydimethylsiloxanes, polyamide, polystyerene, ethylene methacrylate, PTFE, cellulose triacetate and mixtures thereof.

The protective composition used in the method and comprised within the kit according to the invention may additionally comprise a sensate. Sensate materials may provide a sensible signal, such as a cooling or heating sensation, to the user through the skin indicating the presence of the protective composition according to the invention. Such a signal may reinforce the tactile and/or visible signal provided by the powder. Non-limiting examples of sensates include menthol, methone glycerin acetal, methyl diisopropyl propionamide, menthyl lactate, menthoxy-1,2-propanediol, ethyl carboxamide, trimethyl butanamide, menthyl lactate, butanedioic acid monomenthyl ester, vanillyl butyl ether, 1-isopulegol, cineole, methylsalicylate, eucalyptus oil, carvone, rosemary oil, ginger oil, clove oil, ethyl menthane, carboxamide, camphor, eucolytol, peppermint oil, and combinations thereof. If present, the protective composition may comprise from 0.01% to 2.0%, preferably from 0.01 % to 1.0% of sensate, by weight of the protective composition.

Any depilatory composition comprising a suitable keratin reducing agent may be used in the present method and included in the present kit. Non-limiting examples of suitable keratin reducing agents include: sulphide salts such as $Li_2S$, $Na_2S$, $K_2S$, MgS, CaS, SrS or BaS, hydrogen sulphide salts such as NaSH or KSH; thioglycol; thioglycerol; thioglycolamide; thioglycolhydrazide; thioglycolic acid; thioglycolate salts (such as potassium thioglycolate, calcium thioglycolate, ammonium thioglycolate, diammonium dithioglycolate, glyceryl monothioglycolate, or monoethanolamine thioglycolate); thiosalicylic acid; thiomalic acid; ammonium thiolactate; monoethanolamine thiolactate; dithioerythritol; 2-mercaptopropionic acid; 1,3-dithiopropanol; glutathione; dithiothreitol; cysteine; homocysteine; N-acetyl-L-cysteine and cysteamine. Advantageously, the keratin reducing agent is comprised within the depilatory composition in an amount from 0.3% to 20%, preferably from 0.8% to 15%, more preferably from 1% to 10% by weight of the depilatory composition.

Advantageously, the depilatory composition may comprise at least one thioglycolate salt or thioglycollic acid acting as a hair removal agent when the depilatory composition is applied to unwanted hair. Preferably, the depilatory composition comprises sodium, potassium, magnesium, calcium, beryllium, strontium, zinc, monoethanolamine, ammonium, tetralkylammonium, imidazolium, pyridinium, phosphonium or glyceryl thioglycolate salts, or mixtures thereof, which may include dianion forms of thioglycolate. More preferably, the depilatory composition comprises at least one of sodium, potassium, magnesium or calcium thioglycolate, or mixtures thereof. Even more preferably the depilatory composition comprises potassium or calcium thioglycolate, or mixtures thereof.

The pH of the depilatory composition may advantageously be in the range of from 6 to 13.8, preferably from greater than 7 to 13, more preferably from 9 to 12.9, even more preferably from 10 to 12.8, even more preferably still from 12 to 12.75 and yet more preferably from 12.3 to 12.6 to improve the efficacy of the active ingredient. The depilatory composition may, in a preferred embodiment, comprise at least one base to control the pH. Preferably, the depilatory composition comprises potassium hydroxide; sodium hydroxide; lithium hydroxide; calcium hydroxide; barium hydroxide; caesium hydroxide; sodium hydroxide; ammonium hydroxide; strontium hydroxide; rubidium hydroxide; magnesium hydroxide; zinc hydroxide; sodium carbonate; pyridine; ammonia; alkanolamides (including monoethanolamine, diethanolamine, triethanolamine), phosphates (including tetrasodium phosphate), arginine or mixtures thereof. More preferably, the depilatory composition comprises at least one buffering base, even more preferably the depilatory composition comprises calcium hydroxide, magnesium hydroxide; barium hydroxide; strontium hydroxide; zinc hydroxide; arginine or mixtures thereof. Still more preferably the depilatory composition comprises calcium hydroxide; magnesium hydroxide, zinc hydroxide, sodium hydroxide, potassium hydroxide or mixtures thereof. Even more preferably still, the depilatory composition comprises calcium hydroxide, sodium hydroxide or mixtures thereof.

In an advantageous embodiment, the base is present at a concentration of from 0.1% to 10.0%, more preferably from 0.5% to 8.0% and even more preferably from 1.0% to 5.0%, by weight of the depilatory composition.

The concentration of water in the depilatory composition is preferably at least 40%, more preferably from 50% to 98%, even more preferably from 60% to 95% and even more preferably still from 70% to 90%, by weight of the depilatory composition.

The depilatory composition may optionally comprise a thickening agent. A representative but not exhaustive list can be found in "The Encyclopaedia of Polymers and Thickeners for Cosmetics" compiled and edited by Robert Y. Lochhead, PhD and William R. Fron, Department of Polymer Science, University of Southern Mississippi. Exemplary classes of thickening agents include gums, carbomers, polymers and copolymers of acrylic acid, associated thickeners, layered silicates/clays and natural polymers (including polysaccharides). One or more thickening agents may be included in the aqueous depilatory composition. The thickening agent may be present at a level of from about 0.01% to about 20%, preferably from about 0.1% to about 10% by weight of the depilatory composition.

The depilatory composition may also include other skin care ingredients such as conditioning agents selected from the group consisting of humectants, moisturizers, or skin conditioners (including mineral oil; almond oil; chamomile oil; jojoba oil; avocado oil; shea butter, niacinamide and glycerine); skin rejuvenation compositions (for example targeted for fine lines, wrinkles and uneven skin tone, including retinoids), cosmetic compositions; anti-inflammatory agents (including corticosteroids); anti-oxidants (including flavonoids) radical scavengers; sunscreen agents; skin cooling or warming agents and the like. The depilatory composition may comprise one or more skin care ingredients present in an amount of from about 0.001% to about 10%, more preferably from about 0.01% to about 7%, and even more preferably from about 0.025% to about 5%, by weight of the depilatory composition.

An accelerant may be employed in the depilatory composition. This optional component accelerates the rate of depilatory action of the depilatory agent. Suitable accelerants include, but are not limited to, urea; thiourea; dimethyl isosorbide; arginine salts; ethoxydiglycol; propylene glycol and methylpropyldiol. The accelerant may be present in a concentration range of from 0.5% to 10%, more preferably from 2% to 8% and even more preferably from 2% to 5% by weight of the depilatory composition.

The depilatory composition may further comprise components known, conventionally used, or otherwise effective for use in cosmetic compositions, such as dyes; pigments (including ultra marines and talc); anionic, cationic, non-ionic and/or amphoteric or zwitterionic surfactants, polymers (including hydrophobically modified polymers); dispersing agents; solvents; lubricants; fragrances; preservatives; chelants, proteins and derivatives thereof, plant materials (e.g. aloe, chamomile and henna extracts); silicones (volatile or non-volatile, modified or non-modified); film-forming agents; film forming promoters and mixtures thereof.

The depilatory composition may be formulated in any common delivery form, such as a cream or lotion. Alternatively, it may be delivered on a substrate, such as a thin film of depilatory composition coated onto the substrate. The substrate may be configured in any suitable form, such as a strip, mask or patch.

In addition to the protective composition and the depilatory composition, the kit according to the second aspect of the invention may comprise one or more of:

(a) A make-up removal composition and/or a make-up removal wipe;

(b) Means for removal of the protective composition and the depilatory composition following use, which means may comprise one or more of a tool, such as a scraper or a spatula; or a wipe;

(c) A post-treatment composition skin care composition to be applied to the area of skin from which hair has been removed. Such a post-treatment skin care composition may comprise ingredients to promote skin conditioning; moisturizers, skin rejuvenation compositions (targeted for fine lines, wrinkles and uneven skin tone, for example), cosmetic compositions (e.g., foundation, rouge), sunscreens and the like. The post-treatment skin care composition may be leave-on or a rinse-off composition.

(d) Instructions regarding how to use the various elements of the kit, which instructions may comprise one or more elements of the method as defined herein.

Prior to applying the method or using the kit according to the present invention, a user should advantageously remove all make-up from the skin, to ensure good adherence and effective application of both the protective composition and the depilatory composition.

The method according to the first aspect of the invention comprises the step of applying the above-defined protective composition to an area of skin on which unwanted hair is growing. The area of skin may be located on any part of the human body.

Advantageously, the protective composition is not just applied to the area to be depilated, but also to an immediately juxtaposing area thereabout (that is, the protective composition is applied to an area of skin which is greater than just the area which is to be depilated).

Advantageously, the user will apply from 0.3 - 2mg of protective composition per square centimetre of skin, preferably from 0.4 - 1mg/cm$^2$, more preferably from 0.4 to 0.7mg/cm$^2$.

Following application, the protective composition is advantageously massaged into the skin. Preferably, massaging is effected for at least 10 seconds, and, more preferably, massaging is effected as a circular motion. Without wishing to be bound by theory, it is believed that the protective composition may trap hair within it thereby shielding it from the to-be-applied depilatory composition; massaging may help to release the hairs from the skin and ensure improved access thereto by the depilatory composition.

The method according to the first aspect of the invention comprises the subsequent step of applying the above-defined depilatory composition to an area of skin on which unwanted hair is growing and to which protective composition has already been applied. Advantageously, the user will apply a layer of depilatory composition which is from 0.1mm to 5mm, preferably from 0.3 to 3mm, more preferably from 0.5 to 2mm in thickness.

Subsequently, according to the method of the first aspect of the invention, the depilatory composition is advantageously left in place for at least 1 minute, preferably from 1 to 10 minutes, more preferably from 3 to 10 minutes, depending on the thickness of the hair and the hair removal efficacy of the depilatory composition (which, in turn, is dependent upon the concentration of keratin reducing agent in the depilatory composition).

Subsequently, according to the method of the first aspect of the invention, the protective composition and the depilatory composition are advantageously removed. This may be achieved using one or more of a cotton wool ball, pad or wand, a tissue, a cloth, or a tool, such as a spatula or a scraper. Advantageously, the skin from which hair has been removed is then rinsed with water.

In an advantageous subsequent step, a post-treatment skin care composition may be applied to the area of skin from which hair has been removed. Such a post-treatment skin care composition may comprise ingredients to promote skin conditioning; moisturizers, skin rejuvenation compositions (targeted for fine lines, wrinkles and uneven skin tone, for example), cosmetic compositions (e.g., foundation, rouge), sunscreens and the like. The post-treatment skin care composition may be leave-on or a rinse-off composition.

Franz cell method

Principle and Scope:

This method is applicable for using Franz cell apparatus for the *in-vitro* assessment of penetration of thioglycolic acid (TGA) and its salts through a skin mimic after the application of a depilatory composition following pre-treatment with a protective composition.

Penetrated TGA is quantified using Reverse Phase High Performance (or Pressure) Liquid Chromatography (RP-HPLC) with external standard quantitation at 240nm.

Method

Reference is made to Figure 1 and to the reference numerals therein:

1. Prepare the Vitro-Skin (IMS Vitro-Skin®, Catalogue number: P&G1013, made by IMS Inc., Portland, Maine, USA) samples by cutting 8x6.2cm segments and placing them textured side up on the racks into a hydration chamber (manufactured & sold by IMS) containing a 14.7% glycerol solution. The hydration chamber should be sealed and the vitro-skin left to hydrate at room temperature and a humidity of 80.4% $\pm$3.5% for 24 hours.

2. Prepare the receptor solution for the Franz-cell by mixing 1.90ml formic acid (98%wt+ Fluka, by Sigma Aldrich, or equivalent), 30ml acetonitrile (RP-HPLC grade) and 968.1ml water (RP-HPLC grade). Set up the static Franz cell (Permegear or equivalent, 15mm diameter unjacketed cell with a 12ml receptor volume) by clamping it in place over suitable stirrer plates (not shown) and add a small stirrer bar (6) to each cell, fill the receptor cell (2) to the brim with the required amount of receptor solution.

3. Once hydrated, remove a sheet of vitro-skin from the hydration chamber and lay textured side up on a clean flat surface then dose 100$\mu$l ($\sim$2mg/cm$^2$) of protective composition (not shown) onto the vitro-skin and spread evenly over the surface by rubbing for 30 seconds with a gloved finger.

4. Using a scalpel blade cut the vitro-skin segment (3) into two equal sections, each large enough to completely cover the top of the cell. Place the relevant size o-ring (5) (22mm, for the specified Franz-cell) onto each section of the vitro skin and dose to 150mg/cm$^2$ of depilatory composition (4) ("Veet Normal Skin Hair Removal Cream" or an equivalent (an equivalent being a composition comprising 3.7%wt thioglycolic acid)) into the centre then, using a glass rod, evenly spread the cream around the inside of the o-ring (5). Using tweezers pick up the vitro-skin segment and place the vitro-skin segment, depilatory and o-ring centrally over the receptor cell (2), place donor cell (1) over the top and clamp in place. Turn on stirrer plate and start 10 minute countdown timer. After 10 minutes; turn off stirrer and remove the clamp, donor cell (1) and vitro-skin segment and place the receptor solution in a suitable container for analysis.

5. A reference sample should also be run without protective composition treatment on the vitro-skin. Remove a sheet of vitro-skin from the hydration chamber and lay textured side up on a clean flat surface. Repeat step 4 of the protocol to produce the reference sample.

Sample Analysis

For RP-HPLC analysis, prepare a 50mM Formic acid (98%+ Fluka) solution and mix 970ml of this solution with 30ml acetonitrile (HPLC grade) to act as a mobile phase during the analysis.

A reference standard solution should be made with a concentration of Calcium Thioglycolate Trihydrate of 0.94 mg/ml.

Install a Waters Atlantis T3 3$\mu$m 4.6 x 50mm column into the HPLC (although any silica- based C$_{18}$ reversed phase RP-HPLC column may be used), and ensure all solvent lines for the RP-HPLC are primed and free of leaks. Allow the mobile phase to circulate through the system for 25 minutes at 0.7mL/Min in order to equilibrate the column. Detection of the thioglycolic acid is via UV spectroscopy.

The RP-HPLC conditions are as follows:

- Injection volume: 20 $\mu$L

- Mobile phase flow rate: 0.70 ml/min

- Run time: 10 minutes

- UV Detection wavelength: 240 nm

- Column temperature: 35°C

- UV sampling rate: ≥ 5 per second

- Retention time: Thioglycolic Acid ~ 2.5 min

Calculations:

Calculate the concentration of Thioglycolic Acid in the sample

$$\text{concentration (mg/ml)} = \frac{\textbf{weight of std (mg)} \times \textbf{purity}}{25} \times \frac{\textbf{3}}{25}$$

Calculate the concentration of thioglycolic acid in the sample using the following formula:

$$\text{concentration (mg/ml)} = \textbf{A/B} \times \textbf{C} \times \textbf{E/F}$$

Where,

A = Peak Area of Thioglycolic Acid Sample
B = Average Peak Area of Thioglycolic Acid Standard
C = Thioglycolic Acid final STD concentration in mg/ml (0.94 mg/ml)
E = Molecular weight of Thioglycolic acid (92.12g/mol)
F = Molecular weight of Calcium thioglycolate (184.23g/mol)

The efficacy of the barrier provided by the protective composition (resistance to TGA penetration) can be calculated as a percentage decrease in TGA in the receptor solution:

$$\%\text{reduction} = \frac{\text{concentration without barrier* – concentration with barrier*}}{\text{concentration without barrier*}} \times 100$$

*protective composition

For example, if TGA in solution without protective composition = 75µg/ml and TGA in solution with barrier = 15 µg/ml

$$\%\text{reduction} = \frac{75 - 60}{75} \times 100 = \textbf{85\%}$$

A reduction of TGA penetration of 45% or more is believed to correlate to a significant and user-noticeable reduction in irritation.

Examples

| Example | Protective Composition | % Reduction in Thioglycolic Acid Penetration According to the Franz Cell Method |
|---|---|---|
| Example 1 | 100% petrolatum with cone penetration of 170 | 61.0 |
| Comparative Example 1 | 100% Petrolatum with cone penetration of 240 | 41.0 |
| Comparative Example 2 | 100% Mineral oil | 25.0 |
| Comparative Example 3 | 100% Sunflower Seed oil | 10.8 |
| Comparative Example 4 | 100% Olive oil | 0.0 |

As demonstrated by the Franz Cell data, oils, on their own (see Comparative Examples 2-4) provide little to no barrier to thioglycolic acid and even petrolatum with a cone penetration outside the defined range provides a relatively poor barrier, whereas a petrolatum having a cone penetration in the defined range gives a dramatically superior barrier to penetration (see the Example).

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

**Claims**

1.   A method of removing hair from skin, comprising the steps of:

   (a) applying a protective composition to an area of skin on which unwanted hair is growing, wherein the protective composition comprises at least 75% petrolatum by weight of the protective composition, the protective composition having a cone penetration of 160 to 210;
   (b) applying a depilatory composition to the area of skin to which the protective composition has been applied, the depilatory composition comprising a keratin reducing agent.

2.   The method of claim 1, wherein the protective composition comprises at least 80%, preferably at least 90% and more preferably 95% petrolatum by weight of the protective composition.

3.   The method of claim 1 or 2, wherein the protective composition comprises from 1% to 25%, preferably from 2% to 15%, more preferably from 5% to 10% of powder by weight of the protective composition.

4.   The method of any one of claims 1 to 3, wherein the partcilces comprised within the powder have an average particle size from $0.05\mu m$ to $200\mu m$, preferably from $0.1\mu m$ to $130\mu m$, more preferably from $0.2\mu m$ to $60\mu m$.

5.   The method of any of preceding claim, comprising the following additional step between step (a) and step (b):

   (a1) massaging the protective composition into the skin for at least 10 seconds.

6.   The method of any preceding claim, comprising the following additional step immediately following step (b):

   (c) leaving the depilatory composition in place on the protective composition for a period of at least 1 minute, preferably from 1 to 10 minutes, more preferably from 3 to 10 minutes

7.   The method of any preceding claim, wherein the keratin reducing agent comprises a thioglycolate salt, preferably potassium or calcium thioglycolate, or mixtures thereof.

8.   A depilatory kit comprising:

(a) a protective composition, the protective composition comprising at least 75% petrolatum by weight of the protective composition, the protective composition having a cone penetration of 160 to 2 10;
(b) a depilatory composition comprising an effective amount of a keratin reducing agent.

9. The kit of claim 8, wherein the protective composition comprises at least 80% preferably at least 90% and more preferably 95% petrolatum by weight of the protective composition.

10. The depilatory kit of claim 8 or 9, wherein the protective composition comprises from 1% to 25%, preferably from 2% to 15%, more preferably from 5% to 10% of powder by weight of the protective composition.

11. The depilatory kit of any of claims 8 to 10, wherein the powder has an average particle size from $0.5\mu$m to $200\mu$m, preferably from $1\mu$m to $130\mu$m, more preferably from $5\mu$m to $30\mu$m.

12. The depilatory kit of any of claims 8 to 11, additionally comprising a tool, such as a scraper or a spatula, or a wipe.

13. The depilatory kit of any of claims 8 to 12, additionally comprising instructions: to use the kit.


**Patentansprüche**

1. Verfahren zum Entfernen von Haaren von Haut, das folgende Schritte umfasst:

(a) das Auftragen einer Schutzzusammensetzung auf einen Hautbereich, auf dem unerwünschter Haarwuchs vorhanden ist, wobei die Schutzzusammensetzung mindestens 75 Gew.-% Petrolat bezogen auf das Gesamtgewicht der Schutzzusammensetzung umfasst, wobei die Schutzzusammensetzung eine Kegelpenetration von 160 bis 210 aufweist;
(b) das Auftragen einer Enthaarungszusammensetzung auf den Hautbereich, auf den die Schutzzusammensetzung aufgetragen wurde, wobei die Enthaarungszusammensetzung ein Keratinreduktionsmittel umfasst.

2. Verfahren nach Anspruch 1, wobei die Schutzzusammensetzung mindestens 80 Gew.-%, vorzugsweise mindestens 90 Gew.-% und mehr bevorzugt 95 Gew.-% Petrolat bezogen auf das Gesamtgewicht der Schutzzusammensetzung umfasst.

3. Verfahren nach Anspruch 1 oder 2, wobei die Schutzzusammensetzung 1 Gew.-% bis 25 Gew.-%, vorzugsweise 2 Gew.-% bis 15 %, mehr bevorzugt 5 Gew.-% bis 10 Gew.-% Puder bezogen auf das Gesamtgewicht der Schutzzusammensetzung umfasst.

4. Verfahren nach einem der Ansprüche 1 bis 3, wobei die im Puder enthaltenen Partikel eine durchschnittliche Partikelgröße von 0,05 $\mu$m bis 200 $\mu$m, vorzugsweise von 0,1 $\mu$m bis 130 $\mu$m, mehr bevorzugt von 0,2 $\mu$m bis 60 $\mu$m aufweisen.

5. Verfahren nach einem der vorstehenden Ansprüche, das folgenden zusätzlichen Schritt zwischen Schritt (a) und Schritt (b) umfasst:

(a1) das Einmassieren der Schutzzusammensetzung in die Haut mindestens 10 Sekunden lang.

6. Verfahren nach einem der vorstehenden Ansprüche, das unmittelbar nach Schritt (b) den folgenden zusätzlichen Schritt umfasst:

(c) das Belassen der Enthaarungszusammensetzung auf der Schutzzusammensetzung für einen Zeitraum von mindestens 1 Minute, vorzugsweise von 1 bis 10 Minuten, mehr bevorzugt von 3 bis 10 Minuten.

7. Verfahren nach einem der vorstehenden Ansprüche, wobei das Keratinreduktionsmittel ein Thioglycolatsalz, vorzugsweise Kalium- oder Calciumthioglycolat, oder Mischungen davon umfasst.

8. Enthaarungskit, das Folgendes umfasst:

(a) eine Schutzzusammensetzung, wobei die Schutzzusammensetzung mindestens 75 Gew.-% Petrolat bezogen auf das Gesamtgewicht der Schutzzusammensetzung umfasst, wobei die Schutzzusammensetzung eine Kegelpenetration von 160 bis 210 aufweist;
(b) eine Enthaarungszusammensetzung, die eine wirksame Menge eines Keratinreduktionsmittels umfasst.

**9.** Kit nach Anspruch 8, wobei die Schutzzusammensetzung mindestens 80 Gew.-%, vorzugsweise mindestens 90 Gew.-% und mehr bevorzugt 95 Gew.-% Petrolat bezogen auf das Gesamtgewicht der Schutzzusammensetzung umfasst.

**10.** Enthaarungskit nach Anspruch 8 oder 9, wobei die Schutzzusammensetzung 1 Gew.-% bis 25 Gew.-%, vorzugsweise 2 Gew.-% bis 15 %, mehr bevorzugt 5 Gew.-% bis 10 Gew.-% Puder bezogen auf das Gesamtgewicht der Schutzzusammensetzung umfasst.

**11.** Enthaarungskit nach einem der Ansprüche 8 bis 10, wobei das Puder eine durchschnittliche Partikelgröße von 0,5 $\mu$m bis 200 $\mu$m, vorzugsweise von 1 $\mu$m bis 130 $\mu$m, mehr bevorzugt von 5 $\mu$m bis 30 $\mu$m aufweist.

**12.** Enthaarungskit nach einem der Ansprüche 8 bis 11, das außerdem ein Werkzeug wie einen Schaber oder einen Spachtel oder ein Wischtuch umfasst. 10

**13.** Enthaarungskit nach einem der Ansprüche 8 bis 12, das zusätzlich Anweisungen für die Anwendung des Kits umfasst.

## Revendications

**1.** Procédé d'élimination de poils de la peau, comprenant les étapes consistant à :

(a) appliquer une composition protectrice sur une zone de peau sur laquelle poussent des poils indésirables, dans lequel la composition protectrice comprend au moins 75 % de pétrolatum en poids de la composition protectrice, la composition protectrice présentant une pénétration de cône de 160 à 210 ;
(b) appliquer une composition dépilatoire sur la zone de peau sur laquelle la composition protectrice a été appliquée, la composition dépilatoire comprenant un agent réducteur de kératine.

**2.** Procédé selon la revendication 1, dans lequel la composition protectrice comprend au moins 80 %, de préférence au moins 90 % et plus préférablement 95 % de pétrolatum en poids de la composition protectrice.

**3.** Procédé selon la revendication 1 ou 2, dans lequel la composition protectrice comprend de 1 % à 25 %, de préférence de 2 % à 15 %, plus préférablement de 5 % à 10 % de poudre en poids de la composition protectrice.

**4.** Procédé selon l'une quelconque des revendications 1 à 3, dans lequel les particules comprises au sein de la poudre ont une taille moyenne de particules allant de 0,05 $\mu$m à 200 $\mu$m, de préférence de 0,1 $\mu$m à 130 $\mu$m, plus préférablement de 0,2 $\mu$m à 60 $\mu$m.

**5.** Procédé selon l'une quelconque des revendications précédentes, comprenant l'étape supplémentaire suivante entre l'étape (a) et l'étape (b) :

(a1) masser la composition protectrice dans la peau pendant au moins 10 secondes.

**6.** Procédé selon l'une quelconque des revendications précédentes, comprenant l'étape supplémentaire suivante immédiatement après l'étape (b) :

(c) laisser la composition dépilatoire en place sur la composition protectrice pendant une période d'au moins 1 minute, de préférence de 1 à 10 minutes, plus préférablement de 3 à 10 minutes.

**7.** Procédé selon l'une quelconque des revendications précédentes, dans lequel l'agent réducteur de kératine comprend un sel de thioglycolate, de préférence du thioglycolate de potassium ou calcium, ou leurs mélanges.

**8.** Trousse dépilatoire comprenant :

(a) une composition protectrice, la composition protectrice comprenant au moins 75 % de pétrolatum en poids de la composition protectrice, la composition protectrice présentant une pénétration de cône de 160 à 210 ;

(b) une composition dépilatoire comprenant une quantité efficace d'un agent réducteur de kératine.

9. Trousse selon la revendication 8, dans laquelle la composition protectrice comprend au moins 80 %, de préférence au moins 90 % et plus préférablement 95 % de pétrolatum en poids de la composition protectrice.

10. Trousse dépilatoire selon la revendication 8 ou 9, dans laquelle la composition protectrice comprend de 1 % à 25 %, de préférence de 2 % à 15 %, plus préférablement de 5 % à 10 % de poudre en poids de la composition protectrice.

11. Trousse dépilatoire selon l'une quelconque des revendications 8 à 10, dans laquelle la poudre a une taille moyenne de particules allant de 0,5 $\mu$m à 200 $\mu$m, de préférence de 1 $\mu$m à 130 $\mu$m, plus préférablement de 5 $\mu$m à 30 $\mu$m.

12. Trousse dépilatoire selon l'une quelconque des revendications 8 à 11, comprenant en outre un outil, tel qu'un racloir ou une spatule, ou une lingette.

13. Trousse dépilatoire selon l'une quelconque des revendications 8 à 12, comprenant en outre des instructions pour utiliser la trousse.

EP 2 517 692 B1

# Fig.1.

12

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4401663 A
- US 4424205 A

- US 20040219118 A

**Non-patent literature cited in the description**

- Standard Test Method for Cone Penetration of Petrolatum. ASTM International, 2003

- Standard Test Methods for Cone Penetration of Lubricating Grease. ASTM International, 2003